# EUROPÄISCHE PATENTSCHRIFT

(11) **EP 4 337 105 B1**
(45) Veröffentlichungstag und Bekanntmachung des Hinweises auf die Patenterteilung: **08.10.2025**
(21) Anmeldenummer: 22724417.5
(22) Anmeldetag: 10.05.2022
(51) Int. Cl.: A61B 10/02, A61B 10/04, A61B 5/00, A61B 1/04, A61B 10/00, A61B 1/06, A61B 1/00

(54) **BIOPSIESYSTEM**
BIOPSY SYSTEM
SYSTÈME DE BIOPSIE

(30) Priorität: 14.05.2021 DE 102021204907
(43) Veröffentlichungstag der Anmeldung: 20.03.2024
(73) Patentinhaber: Richard Wolf GmbH, 75438 Knittlingen (DE)
(72) Erfinder: WEBER, Bernd Claus, 76228 Karlsruhe (DE); HÄHNLE, Friedrich, 75015 Bretten (DE); LAMBERTZ, Matthias, 75015 Bretten (DE); HÜTTENBERGER, Dirk, 66887 Rammelsbach (DE)
(74) Vertreter: Patentanwälte Hemmer Lindfeld Frese Partnerschaft mbB
(86) Internationale Anmeldenummer: PCT/DE2022/200096
(87) Internationale Veröffentlichungsnummer: WO 2022/237942

(56) Entgegenhaltungen:
- CN-A- 109 674 438
- US-A1- 2005 203 419
- US-A1- 2009 326 384
- US-A1- 2010 168 610
- US-A1- 2017 319 186

## Beschreibung

Die vorliegende Offenbarung betrifft ein Biopsiesystem zur Entnahme von Gewebeproben eines organischen, vorzugsweise menschlichen oder tierischen, Körpers, insbesondere zur zytologischen Untersuchung. Insbesondere betrifft die vorliegende Offenbarung ein System zur Feinnadelbiospie (FNB) oder Feinnadelaspirationsbiopsie (FNAB).

Häufig wird im Rahmen der Krebsvorsorge oder -nachsorge oder bei einem Krebsverdachtsfall mittels einer Röntgen-, CT- oder UltraschallUntersuchung oder mit Hilfe eines anderen nicht-invasiven bildgebenden Verfahrens eine Abnormität diagnostiziert, die sich beispielsweise als Verschattung im Untersuchungsbild darstellt. Für die Durchführung einer solchen Untersuchung gibt es unterschiedliche Anlässe, z.B. Vorsorge (z.B. Screening der Lunge von Rauchern), Nachsorge (z.B. Untersuchung der Lunge in der Nachsorge eines kolorektalen Karzinoms), oder eine Untersuchung aus einem anderem Anlass (z.B. Untersuchung einer potenziell verletzten Lunge nach einem Unfall mit Zufallsbefund "Rundherd").

Häufig muss der Detektion einer Abnormität eine Behandlung (Therapie) folgen. Voraussetzung für einen gezielten und effizienten therapeutischen Eingriff bzw. die Entscheidung darüber, ob ein solcher therapeutischer Eingriff überhaupt notwendig ist, ist jedoch die Klärung der genauen Ursache der Abnormität im Untersuchungsbild. Die Ursache für die Verschattung kann nämlich völlig unterschiedlicher Art sein, z.B. Tumor, Infektion, Granulom, usw. Unterschiedliche Ursachen erfordern allerdings teilweise völlig unterschiedliche therapeutische Vorgehensweisen (chirurgische Entfernung, Chemotherapie, Strahlentherapie, hormonelle Behandlung, medikamentöse Behandlung, usw.).

Eine solche Klärung (also die Suche nach der Ursache für das abnorme Erscheinungsbild, d.h. für die Verschattung) geschieht über eine ex situ histopathologische oder zytologische Untersuchung des verdächtigen Gewebes. Für eine solche Untersuchung benötigt man ein Stück Gewebe oder einzelne Zellen aus dem Bereich der detektierten Abnormität.

Ein solches Gewebestück oder Gewebezellen werden über eine Gewebeentnahme oder eine Entnahme von Gewebeflüssigkeit, eine sogenannte Biopsie, gewonnen. Für die Durchführung der Gewebeentnahme gibt es prinzipiell unterschiedliche Möglichkeiten. Soll der Vorgang wenig patientenbelastend sein, dann kann die Biopsie beispielsweise perkutan durchgeführt werden (z.B. CT- oder ultraschallgesteuert, um den verdächtigen Bereich, wo die Probe entnommen werden soll, sichtbar zu machen). Dabei wird eine dünne, lange, starre Biopsienadel von außen durch die Haut und das darunterliegende Gewebe in das betroffene Organ zu der Abnormität geschoben, um dort, an oder innerhalb der Abnormität, eine Gewebeprobe zu entnehmen. Üblicherweise kommen bei der perkutanen Vorgehensweise Hohlnadeln unterschiedlicher Form und Ausführung zum Einsatz.

Die mit den üblichen bildgebenden Verfahren (CT, Ultraschall, usw.) gewonnenen Bilder ermöglichen zwar das Sichtbarmachen von suspektem Gewebe. Jedoch ist der Grad der Gewebe-Differenzierung eingeschränkt, d.h. die Spezifität ist begrenzt.

Eine Abnormität als solche hebt sich zwar relativ deutlich - beispielsweise durch einen anderen Farbton (bei der CT- und US-Darstellung durch einen anderen Grauton) - vom umgebenden gesunden Gewebe ab. Sie kann dadurch vergleichsweise gut detektiert und die gewünschte Entnahme von Gewebe vergleichsweise einfach durchgeführt werden, indem das distale Ende der Biopsienadel beispielsweise in das Zentrum der Läsion geführt wird.

Jedoch kann mit den üblichen bildgebenden Verfahren (CT, US, usw.) keine Aussage darüber gemacht werden, wie bzw. in welcher Form das verschattet dargestellte Gewebe verändert ist, beispielsweise gutartig oder bösartig. So kann beispielsweise eine bereits in der Vergangenheit behandelte Läsion in CT-Aufnahmen in der Nachsorge weiterhin als Verschattung erscheinen, obwohl es sich dabei nur um fibrös verändertes, also nichtmalignes Gewebe handelt. Eine Biopsie aus dem Zentrum der Verschattung und die nachfolgende pathologische bzw. zytologische Untersuchung würde diese Diagnose bestätigen.

Ein ggf. immer noch vorhandener Resttumor (aufgrund von beispielsweise unzureichender Erstbehandlung) oder gar eine Tumorneubildung (Rezidiv, neu entstanden nach der Erstbehandlung) könnte im CT- oder US-Bild, vom umgebenden fibrös veränderten und als Verschattung dargestellten Gewebe nicht differenziert werden und bliebe damit unauffällig.

Eine einzelne Biopsie aus dem Zentrum der scheinbar homogenen Abnormität - es gäbe anhand des CT- oder US-Bildes zunächst keinen Anlass, an einer anderen Stelle zu biopsieren - würde also zu einer folgenschweren Falschbewertung führen, weil sie den im CT- oder US-Bild nicht differenzierbaren Resttumor oder die Tumorneubildung nicht mit erfassen würde und dementsprechend die tatsächlich erforderliche Therapie ausbleiben würde.

Mit den üblichen bildgebenden Verfahren (CT, US, usw.) kann zudem nicht gewährleistet werden, dass tatsächlich das gesamte suspekte Gewebe sichtbar gemacht wird. Zusätzlich zur Spezifität ist also auch die Sensitivität begrenzt. Das heißt, dass eine Abnormität, die genügend groß ist und deren Gewebe genügend stark verändert ist, sich zwar relativ deutlich als Verschattung vom umgebenden gesunden Gewebe abhebt. Es kann in dem Fall relativ einfach Gewebe aus diesem Bereich entnommen werden und der weiteren pathologischen Untersuchung zugeführt werden. Aber frühmaligne Veränderungen oder kleine maligne Veränderungen lassen sich oft nicht oder zumindest nicht gut genug mit den üblichen bildgebenden Verfahren (CT, US, usw.) darstellen. Ggf. zusätzlich vorhandene frühmaligne Veränderungen und kleine maligne Veränderungen, die beispielsweise im Randbereich der Abnormität oder gar außerhalb der Abnormität liegen, blieben in der CT- oder US-Darstellung, unsichtbar.

Eine Therapie (auf der Basis des histopathologischen oder zytologischen Ergebnisses von der Biopsie aus dem zentralen Bereich der Verschattung) würde daher auf das Gebiet der Verschattung und einen "Sicherheitsbereich" um die Verschattung herum begrenzt werden. Etwaige frühmaligne und kleine maligne Veränderungen im Randbereich oder außerhalb der Verschattung (im CT- oder US-Bild unsichtbar) würden nur unzureichend oder gar nicht therapiert werden.

Um dieses Problem zu lösen, ist es bekannt, eine Vielzahl von Biopsien vom gesamten Bereich der Abnormität und auch von dem die Abnormität umgebenden Gewebe zu entnehmen, um das Risiko zu reduzieren, dass pathologisch relevantes Gewebe, das der pathologischen bzw. zytologischen Untersuchung zugeführt werden muss, übersehen wird.

Beispielsweise beschreibt die US 2017/319186 A1 ein Biopsiegerät mit einem in einer Hohlnadel geführten Schaft, der eine seitliche Ausnehmung zur Aufnahme einer Biopsieprobe und einen Lichtleiter aufweist. Die US 2005/203419 A1 beschreibt eine Biopsienadel mit einem seitlichen Probenfenster und einem Lichtleiter. Aus der US 2010/168610 A1 ist ein endoskopisches Biopsieverfahren bekannt, bei dem ein optischer Leiter in eine Biopsiezange eingebettet ist. Die US 2009/326384 A1 beschreibt ein Biopsieinstrument mit integriertem Multi-Lichtleiterfaserbündel.

Eine Vielzahl von Biopsien hat allerdings viele Nachteile und belastet den Patienten erheblich. Erschwerend, d.h. weiter patienten-belastend kommt hinzu, dass eine gute (im Sinne einer aussagekräftigen) Gewebeprobe ausreichend groß sein sollte und deshalb die Biopsienadel und direkt damit zusammenhängend auch der Einstichkanal einen ausreichend großen Durchmesser erfordern.

Außerdem ist die Entnahme vieler Gewebeproben zeitaufwändig, was neben der Belastung der Bedienperson und der Behandlungskosten eine weitere Belastung für den Patienten bedeutet, z.B. längere Narkotisierung. Hinzu kommt die mit zunehmender Zeit zunehmende Strahlenbelastung bei einer CT-gestützten Gewebeentnahme. Da die Gewebeproben allesamt histopathologisch oder zytologisch ausgewertet werden müssen, ist eine solche Vorgehensweise auch kostenintensiv.

Es ist in bestimmten Fällen nicht notwendig, eine vollumfängliche histopathologische Untersuchung des Gewebes durchzuführen. Für eine vollumfängliche histopathologische Untersuchung wird typischerweise ein ganzer Zylinder von Gewebe ausgestanzt (Stanzbiopsie) oder ein relativ großes Gewebestück (Schneidbiospie) ausgeschnitten, um genug Gewebematerial für die Untersuchung ex situ zur Verfügung zu haben. Dies ist für manche medizinische Diagnosen allerdings nicht notwendig. Beispielsweise soll manchmal nur die Aussage getroffen werden, ob in einem verdächtigen Bereich, wie etwa einer Verschattung im CT oder US, ausschließlich benignes Gewebe oder auch malignes Gewebe vorhanden ist. Es reicht dann ggf. nur eine feingewebliche zytologische Untersuchung von wenigen einzelnen Zellen. Für solche Untersuchungen wird typischerweise eine weniger traumatische Feinnadelbiospie (FNB) oder Feinnadelaspirationsbiopsie (FNAB) angewandt. Gegenüber der Schneid- oder Stanzbiopsie ist die FNB bzw. FNAB schonender, schmerzfrei und komplikationsärmer (u.a. weniger Blutungen). Eine Sedierung oder gar Anästhesie ist in der Regel nicht erforderlich. Außerdem kann sie, weil sie schonender vorgeht, größere Bereiche erfassen im Sinne eines "Abscannens" des Organs.

Damit die FNB bzw. FNAB schonender, schmerzfrei, komplikationsärmer abläuft, wird bei der FNB bzw. FNAB eine Feinnadel, deren Durchmesser in der Regel kleiner als 1 mm ist, und damit dünner ist als die Grobnadel für die Stanzbiopsie ist, perkutan in den verschatteten Bereich eingeführt. Bei hochviskosen Flüssigkeiten wie Eiter und Blut werden allerdings auch größere Nadeldurchmesser verwendet.

Bei der FNB bzw. FNAB wird nach Erreichen des Punktionszieles, im Falle der FNAB unter Sog, idealerweise fächerförmig punktiert, um Gewebsmaterial aus unterschiedlichen Regionen zu gewinnen. Beim Vor- und Zurückbewegen der Feinnadel werden dabei Zellen mit einer scharfen distalen Kante der Kanüle abgeschilfert und in die Kanüle eingeschoben bzw. eingesaugt.

Durch die fächerförmige Punktierung und durch die Aneinanderreihung vieler Punktionsfächer (ermöglicht durch den Einsatz einer schonenden Feinnadel), d.h. durch die Gewinnung von Zellmaterial aus vielen unterschiedlichen Bereichen aus einem vergleichsweise großen Volumen, kann der suspekte Gewebebereich (also der im CT oder im US als Verschattung erscheinende Bereich) vollständiger untersucht werden. Vollständiger heißt hier, dass die Wahrscheinlichkeit gegenüber der Stanz- oder Schneidbiopsie geringer ist, dass ggf. kleinere und zunächst unscheinbare maligne Areale innerhalb einer ansonsten benignen Verschattung nicht erfasst bzw. biopsiert und pathologisch nicht untersucht werden. Außerdem kann auch zusätzlich relativ einfach, schnell, schonend und komplikationsarm Gewebsmaterial aus dem Randbereich und aus Bereichen außerhalb des suspekten Bereichs (Verschattung) gewonnen werden.

Besonders schwierig wird die FNB bzw. FNAB, wenn nur in kleinen Mengen malignes Gewebe innerhalb einer größeren Verschattung vorhanden ist. Es müssen immer aus allen Bereichen der Verschattung Zellen abgeschilfert werden und entnommen werden. Das bedeutet, dass der suspekte Bereich (die Verschattung) engmaschig, d.h. in einem relativ engen Punktionskegel (leicht veränderte Nadelausrichtung beim Vor- und Zurückbewegen der Nadel pro Einstich), und vollständig, d.h. mit vielen Punktionskegeln (viele Einstiche an verschiedenen Positionen), sein.

Dieses Prozedere ist (wenn eine wirklich zuverlässige Aussage über das Vorhandensein maligner Areale im Bereich der Verschattung und deren Randbereichen getroffen werden soll) mit einem verhältnismäßig hohen zeitlichen Aufwand verbunden, der umso größer ist, je größer das Volumen der Verschattung ist.

Eine aussagekräftige pathologische Bewertung und Beurteilung des Gewebes (benigne oder maligne) kann immer erst im Anschluss, also nach Abschluss der gesamten Gewebeentnahme, ex situ erfolgen. Dies wiederum hat zur Folge, dass eine lokale Zuordnung nicht mehr möglich ist, d.h. eine Aussage aus welchem Bereich (und ggf. Randbereich) der Verschattung ggf. vorhandene maligne Zellen stammen. Dies wäre aber vorteilhaft, wenn z.B. gezielt aus dem malignen Bereich noch eine zusätzliche Schneid- bzw. Stanzbiopsie entnommen werden soll, um so eine genauere, nämlich vollumfängliche histologische Bewertung zu erreichen, um zu erfahren, um was für eine Art malignes Gewebe es sich genau handelt. Außerdem könnte man gezielt nur den malignen Bereich der Verschattung (und nicht unbedingt den gesamten Bereich der Verschattung) therapieren.

Die FNB bzw. FNAB hat außerdem die Schwierigkeit, dass häufig viel Material aus irrelevanten Gewebebereichen (d.h. zu viele benigne Zellen) und zu wenig - möglicherweise sogar kein - Gewebe aus relevanten Bereichen (d.h. zu wenig oder keine malignen Zellen) entnommen wurde. Das Ergebnis kann also durch einen hohen Anteil an irrelevantem Gewebe bis zur Unkenntlichkeit "verwässert" sein, d.h. eine zu geringe Spezifität aufweisen. Eine Wiederholung der FNB bzw. FNAB (Repunktion) ist bei Tumorverdacht statistisch bei einem Drittel der Patienten erforderlich, was zeitaufwändig und teuer ist und den Patienten zusätzlich belastet.

Es ist daher eine Aufgabe der vorliegenden Offenbarung, ein Biopsiesystem bereitzustellen, welches es erlaubt, mit weniger Biopsien schneller, kostengünstiger und patientenschonender mit höherer Spezifität und höherer Sensitivität zu einer Diagnose zu kommen.

Gemäß der vorliegenden Offenbarung wird zur Lösung dieser Aufgabe ein Biopsiesystem nach Anspruch 1 bereitgestellt.

Mit einem solchen Biopsiesystem kann das Gewebe bereits vor der tatsächlichen Entnahme für die zytologische Untersuchung in situ vorselektiert werden. Das bedeutet, dass das mit den bekannten nichtinvasiven bildgebenden Verfahren (CT, US, usw.) undifferenziert suspekt erscheinende Gewebe bereits in situ differenzierter (z.B. hinsichtlich der Malignität) bewertet werden kann, wodurch die Spezifität erhöht wird. Durch das Fluoreszenz-Endoskopieelement kann Gewebe mit Anregungslicht in situ zur Fluoreszenz angeregt werden. Dabei kann das mittels Anregungslicht angeregte pathologische Gewebe selbst oder eine auf pathologisches Gewebe hinweisende Bakterienansammlung spezifisch fluoreszieren und so gegenüber dem umliegenden gesunden Gewebe erkennbar lokalisiert werden. Die Fluoreszenz-Endoskopie kann beispielsweise mittels eines Photosensibilisators bzw. Markerstoffs (z.B. Chlorin e6) durchgeführt werden, der sich selektiv an pathologischem Gewebe anreichert und fluoresziert.

Mit dem Fluoreszenz-Endoskopieelement kann also sehr genau tatsächlich pathologisches Gewebe lokalisiert werden. Wenn man mittels des Fluoreszenz-Endoskopieelements anhand der Fluoreszenz für die Biopsie relevantes Gewebe lokalisiert hat, kann das Fluoreszenz-Endoskopieelement aus der Einführhülse herausgezogen werden, wobei die Einführhülse im Körper verbleibt. Die Biopsienadel kann dann durch die Einführhülse genau an die Stelle geführt werden, wo das Fluoreszenz-Endoskopieelement das zu untersuchende Gewebe erkannt hat. Es können also mit diesem Biopsiesystem in situ mehr, idealerweise alle relevanten, z.B. frühmalignen und malignen, Bereiche im betreffenden Organ lokalisiert werden, ohne dafür patientenbelastend willkürliche Zufallsbiopsien von nicht relevantem Gewebe entnehmen zu müssen. Das bedeutet eine erheblich höhere Sensitivität.

Die Anzahl der Repunktionen wird mit diesem Biopsiesystem stark reduziert, was die Diagnose deutlich schneller, patientenschonender und kostengünstiger macht. Dies liegt daran, dass die Spezifität der Gewebeprobe in situ bereits geprüft werden kann und somit "verwässerte" Proben mit einem zu großen Anteil an nicht relevantem Gewebe gar nicht erst entnommen werden.

Ein weiterer Vorteil dieses Biopsiesystems ist es, dass damit das Risiko verringert wird, dass die entnommene Gewebeprobe, die pathologisch oder zytologisch als relevantes Gewebestück untersucht wird und über die im Anschluss erfolgende therapeutische Vorgehensweise entscheidet, aus einem benignen, pathologisch irrelevanten Gewebebereich stammt.

Vorzugsweise ist das Biopsiesystem ein System zur Feinnadelbiopsie oder Feinnadelaspirationsbiopsie, wobei das Biopsieelement eine Biopsie-Feinnadel ist. Erfindungsgemäß dient der distale Abschnitt des Fluoreszenz-Endoskopieelements als Saugkolben im Biopsieelement, um Gewebematerial in den Aufnahmeraum zu saugen, wenn der distale Abschnitt des Fluoreszenz-Endoskopieelements von der distalen Position in die proximale Position gebracht wird. Es ist allerdings ebenfalls möglich, dass hier offenbarte Biopsiesystem zur Stanz- oder Schneidbiopsie zu verwenden. Letztlich unterscheidet sich die FNB bzw. FNAB lediglich durch die dünnere Biopsienadel und ggf. durch das Einsaugen von Gewebe in die Biopsienadel von der gröberen Stanz- oder Schneidbiopsie.

Optional kann der distale Abschnitt des Fluoreszenz-Endoskopieelements bei distaler Position den Aufnahmeraum vollständig ausfüllen. Damit kann der distale Abschnitt des Fluoreszenz-Endoskopieelements in einfacher Weise als Saugkolben dienen und das Biopsieelement sehr dünn ausgestaltet werden.

Optional kann das Fluoreszenz-Endoskopieelement dazu eingerichtet sein, wahlweise bei der distalen Position des distalen Abschnitts des Fluoreszenz-Endoskopieelements, der proximalen Position des distalen Abschnitts des Fluoreszenz-Endoskopieelements und/oder einer beliebigen dazwischenliegenden Position des distalen Abschnitts des Fluoreszenz-Endoskopieelements distalseitig Anregungslicht auszukoppeln und Fluoreszenzlicht einzukoppeln. Dies ist besonders interessant, um in situ sofort prüfen zu können, ob sich tatsächlich im Aufnahmeraum malignes Gewebe befindet. Bei distaler Position des distalen Abschnitts des Fluoreszenz-Endoskopieelements wird beispielsweise ein hohes Fluoreszenz-Signal vom Fluoreszenz-Endoskopieelement detektiert und daraufhin zum Einsaugen von Gewebe der distale Abschnitt des Fluoreszenz-Endoskopieelements in die proximale Position gezogen. Wenn sich dabei das Fluoreszenz-Signal abschwächt, bedeutet dies, dass das fluoreszierende Gewebe nicht in den Aufnahmeraum gesaugt wurde. Schwächt sich dagegen das Fluoreszenz-Signal bis zur proximalen Position nicht oder nur wenig ab, so ist dies ein sicheres Zeichen dafür, dass sich fluoreszierendes Gewebe im Aufnahmeraum befindet. Dies kann nicht nur qualitativ, sondern auch quantitativ durch die Höhe des Fluoreszenz-Signals bewertet werden. Es kann also mit einer bestimmten Untergrenze für das Fluoreszenz-Signal sichergestellt werden, dass die Gewebeprobe eine bestimmte Spezifität hat.

Erfindungsgemäß ist der distale Abschnitt des Fluoreszenz-Endoskopieelements radial dichtend in dem Biopsieelement geführt, sodass im Aufnahmeraum während eines proximalwärtigen Zurückziehens des distalen Abschnitts des Fluoreszenz-Endoskopieelements im Biopsieelement ein Einsaugeffekt in den Aufnahmeraum erzeugt wird. Der distale Abschnitt des Fluoreszenz-Endoskopieelement kann also als Saugkolben fungieren.

Optional kann ein distales Ende des distalen Abschnitts des Fluoreszenz-Endoskopieelements in der distalen Position bündig mit einer distalen Spitze des Biopsieelements den Aufnahmeraum abschließen oder distal aus der distalen Spitze des Biopsieelements vorstehen. Dies macht den Einstich und die Vorwärtsbewegung des Biopsieelements schonender in den Bereichen, die gerade nicht biopsiert werden sollen. Der distale Abschnitt des Fluoreszenz-Endoskopieelements kann so lange in der distalen Position verbleiben bis ein ausreichend starkes Fluoreszenz-Signal detektiert wird. Dies schont all das benigne und gesunde Gewebe, das durchstochen werden muss, um zu dem relevanten malignen Gewebe zu gelangen.

Optional kann der distale Abschnitt des Fluoreszenz-Endoskopieelements in der distalen Position und/oder in der proximalen Position relativ zum Biopsieelement arretierbar sein. Eine Arretierung ist zumindest in der distalen Position sinnvoll, damit der distale Abschnitt des Fluoreszenz-Endoskopieelements beim Einstich nicht ungewollt eingeschoben wird oder dabei manuell festgehalten werden muss. Die proximale Position kann durch eine Arretierung ebenfalls festgelegt sein, kann aber auch eine beliebige manuell von der Bedienperson gewählte Axialposition sein. Die Länge des Aufnahmeraums kann entsprechend von der Bedienperson durch die proximale Position gewählt oder durch einen Anschlag oder eine Arretierung festgelegt sein.

Optional kann der distale Abschnitt des Fluoreszenz-Endoskopieelements zumindest in der distalen Position drehfest um seine Längsachse im Biopsieelement in einer definierten Drehstellung gelagert sein. Vorzugsweise ist die Drehstellung durch einen Längssteg in einer entsprechenden Längsnut definiert. Zwischen der distalen Position und der proximalen Position kann die Winkelposition von Längssteg und/oder Längsnut anders sein als in der distalen oder proximalen Position. Dadurch kann eine axiale Arretierung in der distalen oder proximalen Position erzielt werden, wobei durch Drehung des distalen Abschnitts des Fluoreszenz-Endoskopieelements in die andere Winkelstellung die Arretierung gelöst werden kann.

Optional können ein distales Ende des distalen Abschnitts des Fluoreszenz-Endoskopieelements und eine distale Spitze des Biopsieelements in einem gleichen Winkel abgeschrägt sein. Vorzugsweise kann der Winkel spitz sein und vorzugsweise 45° oder weniger betragen.

Optional kann der distale Abschnitt des Fluoreszenz-Endoskopieelements und/oder das Biopsieelement für den einmaligen Gebrauch als Einwegartikel ausgestaltet sein. Dadurch können der distale Abschnitt des Fluoreszenz-Endoskopieelements und/oder das Biopsieelement besonders dünn und günstig hergestellt werden.

Optional kann das Fluoreszenz-Endoskopieelement einen Lichtleiter mit einer proximalseitigen Einkopplung von Anregungslicht und/oder einer proximalseitigen Auskopplung von Fluoreszenzlicht aufweisen, wobei ein distales Ende des Lichtleiters als distalseitige Auskopplung des Anregungslichts und/oder als distalseitige Einkopplung des Fluoreszenzlichts fungiert. Dies ist die bevorzugte Ausführungsform für besonders dünne Biopsieelemente. Der Lichtleiter selbst fungiert als Saugkolben und ist in einem sehr dünnen Kanal des Biopsieelements radial dichtend geführt, sodass ein distaler Abschnitt des Kanals bei zurückgezogenem Lichtleiter den Aufnahmeraum bildet, welcher bei distaler Position vollständig vom Lichtleiter ausgefüllt ist. Der Lichtleiter kann dabei sowohl einen Beleuchtungspfad für das Anregungslicht als auch einen Bildpfad für das Fluoreszenzlicht bilden. "Bildpfad" soll hier nicht darauf beschränkt sein, dass aus dem eingekoppelten Fluoreszenzlicht ein Bild erzeugt wird. Es kann im Bildpfad lediglich ein eingekoppeltes Fluoreszenzsignal übertragen werden, dessen Eigenschaft bestimmt wird.

Optional kann der distale Abschnitt des Fluoreszenz-Endoskopieelements eine LED zur Auskopplung von Anregungslicht und/oder einen Bildsensor, eine Photodiode o.ä. zur Einkopplung von Fluoreszenzlicht aufweisen. In Kombination mit einem Lichtleiter oder alternativ dazu kann dabei der Beleuchtungspfad und/oder der Bildpfad elektrisch zum distalen Abschnitt des Fluoreszenz-Endoskopieelements ausgeführt sein. Es kann eine LED am distalen Ende zur Aussendung des Anregungslichts dienen und das Fluoreszenzlicht entweder über einen Lichtleiter zu einem proximalen Bildsensor, einer Photodiode o.ä. geführt werden oder von einem distalseitigen Bildsensor, einer Photodiode o.ä. aufgenommen werden. Ebenso kann ein Lichtleiter Anregungslischt einer proximalen LED zum distalen Abschnitt des Fluoreszenz-Endoskopieelements führen und dort auskoppeln. Es sei an dieser Stelle angemerkt, dass eine Einkopplung von Fluoreszenzlicht mittels eines Bildsensors, einer Photodiode o.ä. hier nicht unbedingt zur Bilderzeugung verwendet werden muss. Für eine Entscheidung, ob das Fluoreszenzsignal auffällig ist oder nicht, d.h. eine Biopsie entnommen werden soll oder nicht, ist es oft hinreichend, wenn lediglich eine Eigenschaft eines Fluoreszenzsignals, beispielsweise dessen Amplitude oder Intensität, bestimmt wird. Beispielsweise kann sich die Auffälligkeit in einer gegenüber dem umliegenden Gewebe oder gegenüber einem Referenzwert erhöhten oder niedrigeren Fluoreszenz-Intensität zeigen.

Optional kann das Fluoreszenz-Endoskopieelement dazu eingerichtet sein, wahlweise ein erstes Anregungslicht und ein zweites Anregungslicht proximal einzukoppeln, wobei das erste Anregungslicht im Mittel kurzwelliger ist als das zweite Anregungslicht, und wobei das Fluoreszenz-Endoskopieelement dazu eingerichtet ist, wahlweise ein erstes Bild oder ein erstes Fluoreszenzsignal von einem ersten Gewebebereich aus mit dem ersten Anregungslicht angeregtem ersten Fluoreszenzlicht zu erzeugen und ein zweites Bild oder ein zweites Fluoreszenzsignal von einem zweiten Gewebebereich aus mit dem zweiten Anregungslicht angeregtem zweiten Fluoreszenzlicht aufzunehmen. Da die Eindringtiefe von dem kurzwelligen ersten Anregungslicht geringer sein kann als die Eindringtiefe von dem langwelligeren zweiten Anregungslicht, ist der erste Gewebebereich vorzugsweise kleiner als der zweite Gewebebereich. Die Lokalisierung von pathologischem Gewebe erfolgt so mit einer höheren räumlichen Auflösung mittels des ersten Anregungslichts. Da die Eindringtiefe von dem langwelligeren zweiten Anregungslicht größer sein kann, kann auch das detektierte Fluoreszenzsignal aus einem entsprechend größeren zweiten Gewebebereich stammen. Die Lokalisierung von pathologischem Gewebe mittels des zweiten Anregungslichts erfolgt so mit einer geringeren räumlichen Auflösung, es wird aber ein größeres Volumen erfasst. Es kann also zunächst ein größerer zweiter Gewebebereich grobmaschiger mit dem langwelligeren zweiten Anregungslicht vorselektiert werden und dann ein kleinerer erster Gewebebereich, der vorzugsweise ein Teil des zweiten Gewebebereichs ist, engmaschiger mit dem kurzwelligen ersten Anregungslicht untersucht werden.

Optional kann das Fluoreszenz-Endoskopieelement proximalseitig eine mit dem distalen Abschnitt des Fluoreszenz-Endoskopieelements lösbar koppelbare oder fest gekoppelte optische Komponente aufweisen, wobei die optische Komponente einen Strahlteiler mit einer Bildpfadseite und einer Beleuchtungspfadseite aufweist, wobei zum Auskoppeln des Fluoreszenzlichts ein Bildsensor, eine Photodiode o.ä. mit der Bildpfadseite lösbar koppelbar oder fest gekoppelt ist, und wobei zum Einkoppeln des Anregungslichts eine Lichtquelle mit der Beleuchtungspfadseite lösbar koppelbar oder fest gekoppelt ist. Die Lichtquelle ist vorzugsweise mindestens eine LED oder mindestens eine Laserauskopplung. Der Strahlteiler kann beispielsweise ein Prismenpaar aufweisen, das zusammen einen Strahlteilerblock bildet und das Anregungslicht und das Fluoreszenzlicht zumindest zu einem Großteil voneinander trennt, sodass das Fluoreszenzlicht zur Bildpfadseite transmittiert oder reflektiert wird. Entsprechend anders herum kann das Prismenpaar das Anregungslicht zur Beleuchtungspfadseite reflektieren bzw. transmittieren. Vorzugsweise liegen die Bildpfadseite und die Beleuchtungspfadseite des Strahlteilers an in einem Winkel, vorzugsweise von etwa 90°, zueinander angeordneten Seiten des Strahlteilers. Vorzugsweise liegt die Bildpfadseite proximalseitig, sodass Fluoreszenzlicht von der distalen Eintrittsseite des Strahlteilers zur Bildpfadseite im Wesentlichen transmittiert wird. Die Beleuchtungspfadseite liegt vorzugsweise lateralseitig, sodass Anregungslicht von der lateralen Eintrittsseite zur distalen Austrittseite hin im Wesentlichen reflektiert wird.

Optional kann die optische Komponente ein optisches Langpassfilter aufweisen, welches das Fluoreszenzlicht stärker zur Bildpfadseite transmittiert als das Anregungslicht, und/oder ein optisches Kurzpassfilter aufweisen, welches das Fluoreszenzlicht stärker zur Bildpfadseite reflektiert als das Anregungslicht. Beispielsweise kann das Langpassfilter bzw. das Kurzpassfilter zwischen einem als Strahlteiler fungierenden Prismenpaar angeordnet sein und/oder als Schicht auf einer oder beiden der zueinander gewandten Seiten der Prismen realisiert sein. Dies ist insbesondere dann sinnvoll, wenn das Anregungslicht kurzwelliger ist als das Fluoreszenzlicht.

Optional kann das Fluoreszenz-Endoskopieelement eine mit dem Bildsensor, der Photodiode o.ä. und/oder der Lichtquelle signalverbundene oder -verbindbare Betriebseinheit aufweisen, wobei die Betriebseinheit eine optische und/oder akustische Anzeigekomponente, eine Steuer- und Versorgungskomponente und eine Bedienungskomponente aufweist.
Optional kann die optische Komponente in einem Handstück des Fluoreszenz-Endoskopieelements angeordnet sein, an welches der distale Abschnitt des Fluoreszenz-Endoskopieelements und das Biopsieelement ankoppelbar sind, wobei das Handstück vorzugsweise für den mehrmaligen Gebrauch ausgestaltet ist. Dies ist sinnvoll, da die optische Komponente, ggf. mit LED und/oder Bildsensor bzw. Photodiode o.ä., relativ teuer sein kann und das Handstück keinen direkten Patientenkontakt hat. Der distale Abschnitt des Fluoreszenz-Endoskopieelements und das Biopsieelement, welche vorzugsweise Einwegartikel für den einmaligen Gebrauch sind, können dann für die Biopsie aus einer sterilen Verpackung geholt und an das Handstück angekoppelt werden.

Die Begriffe "distalwärtig" bzw. "proximalwärtig" sollen hierin eine relative Position bedeuten, die sich distal bzw. proximal von einer Bedienperson des Systems als Bezugsposition befindet. Die Begriffe "distalseitig" bzw. "proximalseitig" sollen hierin entsprechend Positionen an einer distalen bzw. proximalen Seite eines Gegenstands bedeuten. Die Begriffe "distalwärts" bzw. "proximalwärts" sollen hierin entsprechend Richtungen bedeuten, die sich nach distal bzw. proximal erstrecken.

Die Offenbarung ist nachfolgend anhand von in den Zeichnungen dargestellten Ausführungsbeispielen näher erläutert. Es zeigen:
Fig. 1 schematisch eine beispielhafte Ausführungsform eines hierin offenbarten Biopsiesystems;
Fig. 2-7a-c schematisch die beispielhafte Ausführungsform aus Fig. 1 in jeweils verschiedenen Phasen der Verwendung;
Fig. 8 schematische Schnittdarstellungen einer ersten beispielhaften Ausführungsform einer Arretierung eines hierin offenbarten Biopsiesystems; und
Fig. 9 schematische Schnittdarstellungen einer zweiten beispielhaften Ausführungsform einer Arretierung eines hierin offenbarten Biopsiesystems.

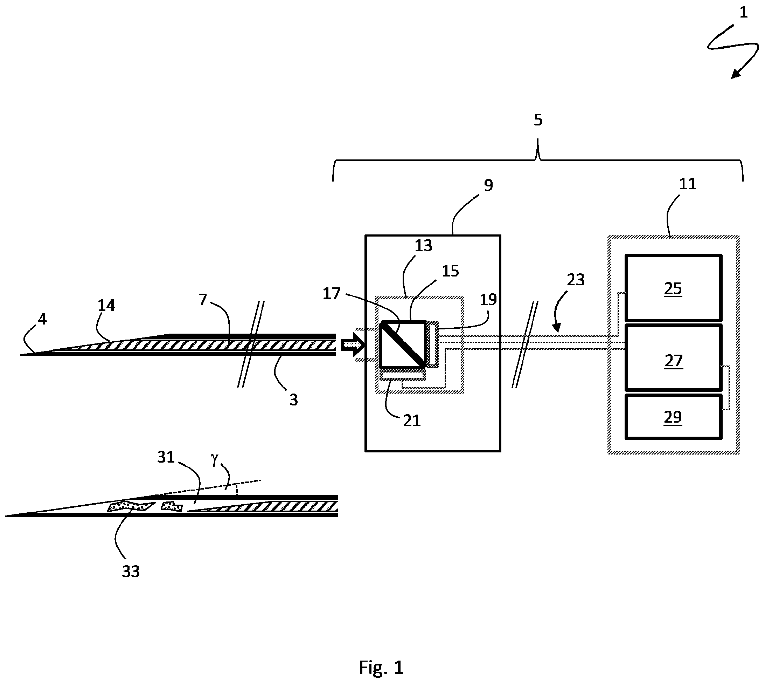

Fig. 1 zeigt ein Biopsiesystem 1 mit einem Biopsieelement 3 in Form einer Biopsie-Feinnadel, die als sehr dünne Kanüle mit einer distalseitig in einem spitzen Winkel γ abgeschrägten distalen Spitze 4. Ferner weist das Biopsiesystem 1 ein Fluoreszenz-Endoskopieelement 5 auf. Das Fluoreszenz-Endoskopieelement 5 selbst weist einen passgenau durch die Kanüle des Biopsieelements 3 geführten distalen Abschnitt 7 in Form eines Lichtleiters, ein Handstück 9 und eine Betriebseinheit 11 auf. In dem Handstück 9 ist eine optische Komponente 13 angeordnet, mit welcher der Lichtleiter 7 lösbar koppelbar oder fest gekoppelt ist. Das Biopsieelement 3 kann zur Handhabung mittels des Handstücks 9 mechanisch an das Handstück 9 lösbar koppelbar oder fest gekoppelt sein. Vorzugsweise ist das Handstück 9 für den mehrmaligen Gebrauch ausgelegt, wogegen das Biopsieelement 3 sowie der Lichtleiter 7 als vormontierte Einheit als Einwegartikel zum einmaligen Gebrauch und zum Ankoppeln an das Handstück 9 ausgelegt sind.

Mit der Ankopplung des Lichtleiters 7 an die optische Komponente 13 wird eine proximalwärtige Auskopplung von Fluoreszenzlicht aus dem Lichtleiter 7 in die optische Komponente 13 sowie eine proximalwärtige Einkopplung von Anregungslicht aus der optischen Komponente 13 in den Lichtleiter 7 erzielt. Ein distales Ende 14 des Lichtleiters ist wie die distale Spitze 4 des Biopsieelements 3 im gleichen spitzen Winkel γ abgeschrägt und kann die Kanüle des Biopsieelements 3 distalseitig bündig abschließen.

Die optische Komponente 13 weist einen Strahlteiler 15 in Form eines Strahlteilerblocks auf, der durch ein Prismenpaar gebildet wird. Die Prismen liegen jeweils mit optischen Grenzflächen aneinander, die sich in einem Winkel von ca. 45° zur optischen Achse erstrecken. Zwischen oder mindestens an oder auf einer der optischen Grenzflächen ist ein Langpassfilter 17 angeordnet, das langwelligeres Fluoreszenzlicht im Wesentlichen transmittiert und kurzwelliges Anregungslicht reflektiert. Im Wesentlichen koaxial zur optischen Achse weist der Strahlteiler 15 daher eine Bildpfadseite auf, an der ein Bildsensor 19, eine Photodiode o.ä. angeordnet ist. Lateral weist der Strahlteiler 15 eine Beleuchtungspfadseite auf, an welcher eine Lichtquelle 21, beispielsweise in Form einer LED oder einer Laserauskopplung, angeordnet ist. Das Langpassfilter 17 reflektiert das Anregungslicht distalwärts zum Lichtleiter 7.

Die Betriebseinheit 11 ist mit dem Bildsensor 19, der Photodiode o.ä. und der Lichtquelle 21 über eine Kabelverbindung 23 signalverbunden oder -verbindbar. Die Lichtquelle 21 kann über die Betriebseinheit 11 gesteuert und die Signale des Bildsensors 19, der Photodiode o.ä. können von der Betriebseinheit 11 empfangen, verarbeitet und ggf. visualisiert werden. Dazu weist die Betriebseinheit 11 eine optische und/oder akustische Anzeigekomponente 25, eine Steuer- und Versorgungskomponente 27 und eine Bedienungskomponente 29 auf.

Die Fig. 1 zeigt das Biopsieelement 3 und den Lichtleiter als distalen Abschnitt 7 des Fluoreszenz-Endoskopieelements 5 in verschiedenen axialen Positionen des Lichtleiters 7 relativ zum Biopsieelement 3. Oben ist der Lichtleiter 7 in einer distalen Position und unten in einer beliebigen proximalen Position. In der unten gezeigten proximalen Position ist der Lichtleiter 7 proximalwärts zurückgezogen, wodurch sich in der Kanüle des Biopsieelements 3 distalseitig vom Lichtleiter 7 ein Aufnahmeraum 31 zur Aufnahme von Gewebeproben 33 ergibt. Alternativ zum Lichtleiter könnte der distale Abschnitt 7 des Fluoreszenz-Endoskopieelements 5 eine distalseitige LED und/oder einen distalseitigen Bildsensor, eine Photodiode o.ä. aufweisen und der Bildpfad und/oder Beleuchtungspfad ohne die optische Komponente 13 elektrisch umgesetzt werden.

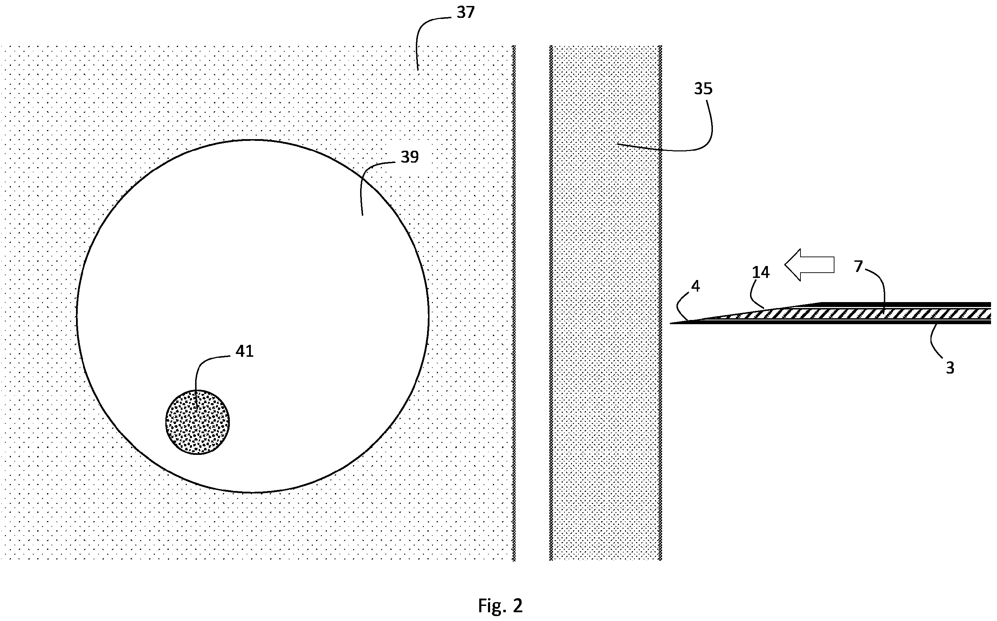

Fig. 2 zeigt das Biopsieelement 3 und den Lichtleiter 7 schematisch in distaler Position kurz vor dem Einstich durch die Haut 35 eines Patienten. Im Körper des Patienten befindet sich ein Organ 37, welches im CT-Bild und/oder US-Bild eine auffällige Verschattung 39 in einem relativ großen Volumen zeigt. Tatsächlich ist in diesem gezeigten Beispiel der größte Teil der Verschattung 39 benignes Gewebe und nur ein kleiner Teil davon malignes Gewebe 41.

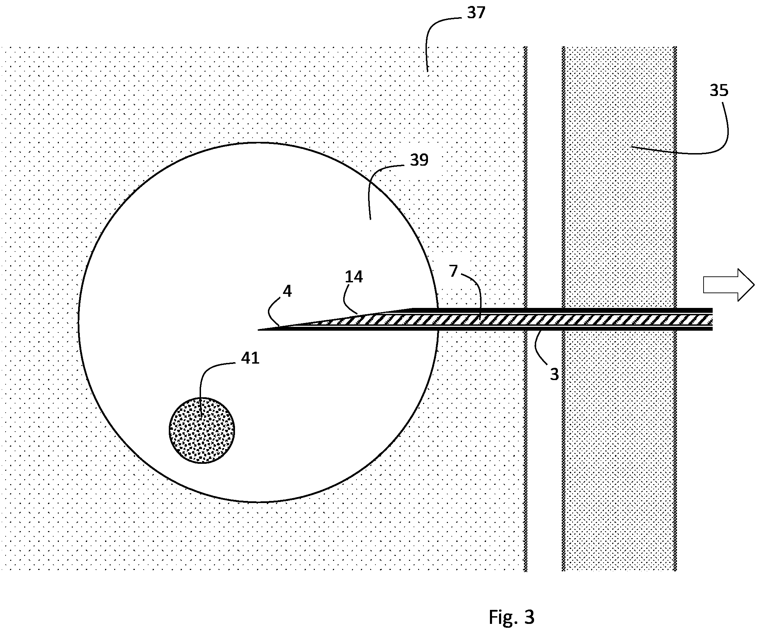

In Fig. 3 ist gezeigt, wie das Biopsieelement 3 mit dem Lichtleiter 7 in arretierter distaler Position in die Verschattung 39 des Organs 37 eingestochen wurde. Der Einstich ist relativ wenig traumatisch, da der Aufnahmeraum 31 durch den Lichtleiter 7 distalseitig verschlossen ist. Dabei wird vorzugsweise permanent Anregungslicht über das distale Ende 14 des Lichtleiters 7 ausgekoppelt. Es wird ebenfalls vorzugsweise ständig überprüft, ob ein Fluoreszenz-Signal empfangen wird. Da der Patient vorzugsweise vor der Biopsie einen Photosensibilisator bzw. Markerstoff (z.B. Chlorin e6) verabreicht bekommen hat, müsste malignes Gewebe vor dem distalen Ende 14 des Lichtleiters 7 auffällig fluoreszieren. Da das benigne Gewebe in der Verschattung 39 nicht oder unauffällig fluoresziert, kann die Bedienperson entscheiden, dass eine Biopsie an dieser Stelle nicht sinnvoll ist und das Biopsieelement 3 und den Lichtleiter 7 wieder zurückziehen.

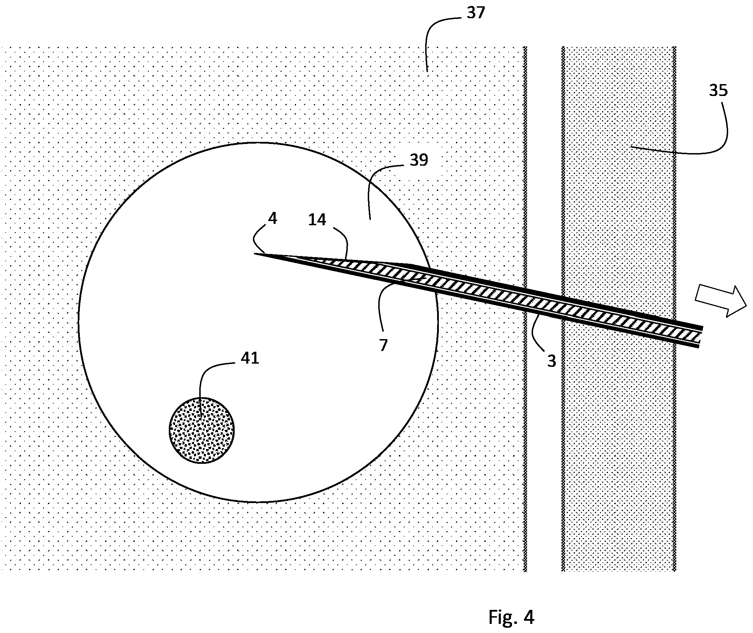

In Fig. 4 ist gezeigt, wie das Biopsieelement 3 mit dem Lichtleiter 7 in arretierter distaler Position unter einem anderen Winkel in die Verschattung 39 des Organs 37 eingestochen wurde. Das Biopsieelement 3 muss dabei ggf. nicht ganz aus der Haut 35 gezogen werden, sondern vielleicht nur aus dem Organ 37 oder der Verschattung 39. Auch dort detektiert das Fluoreszenz-Endoskopieelement 5 kein Fluoreszenz-Signal, da sich vor dem distalen Ende 14 des Lichtleiters 7 nur benignes Gewebe befindet.

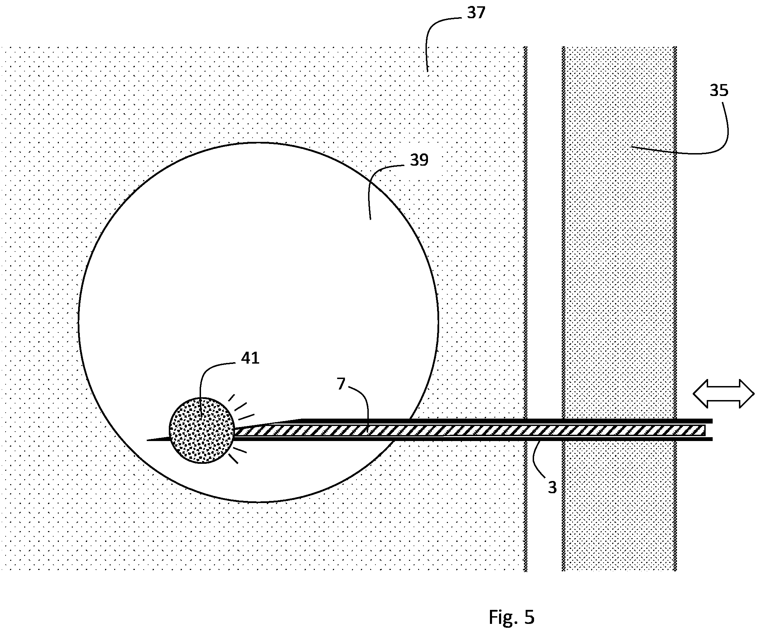

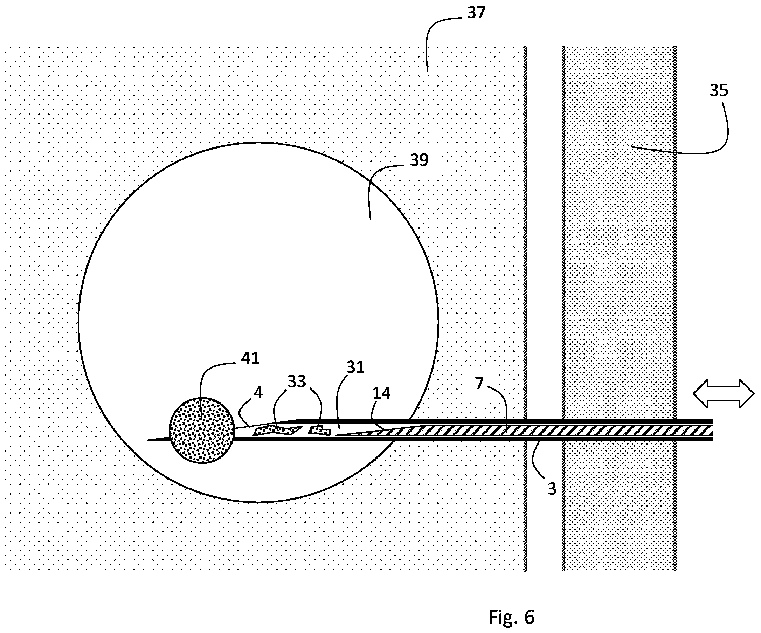

In Fig. 5 hat sich die Bedienperson entschieden, das Biopsieelement 3 mit dem Lichtleiter 7 in arretierter distaler Position ganz neu an anderer Stelle durch die Haut 35 in einen Randbereich der Verschattung 39 zu stechen. Dort befindet sich das maligne Gewebe 41, welches durch Einwirkung des Anregungslichts auffällig fluoresziert. Ist das Fluoreszenz-Signal nach Meinung der Bedienperson auffällig genug, so kann durch Vor- und Zurückbewegen malignes Gewebe 41 abgeschilfert werden (s. Fig. 6). Sodann löst die Bedienperson die Arretierung des Lichtleiters 7 und zieht diesen proximalwärts zurück in eine proximale Position. Die proximale Position kann beliebig sein oder durch einen Anschlag oder eine zweite Arretierung fest vorgegeben sein. Durch das Zurückziehen des Lichtleiters 7 öffnet sich der Aufnahmeraum 31, wobei ein Einsaugeffekt erzeugt wird, mit dem eine vor dem distalen Ende 14 des Lichtleiters 7 liegende abgeschilferte Gewebeprobe 33 vom malignen Gewebe 41 in den Aufnahmeraum 31 eingesaugt wird. Es wird dabei besonders darauf geachtet, dass das Fluoreszenz-Signal auffällig bleibt. Wenn es nämlich stark unauffällig wird, ist das ein klares Zeichen dafür, dass kein malignes Gewebe 41 in den Aufnahmeraum 31 eingesaugt wurde oder zu wenig davon.

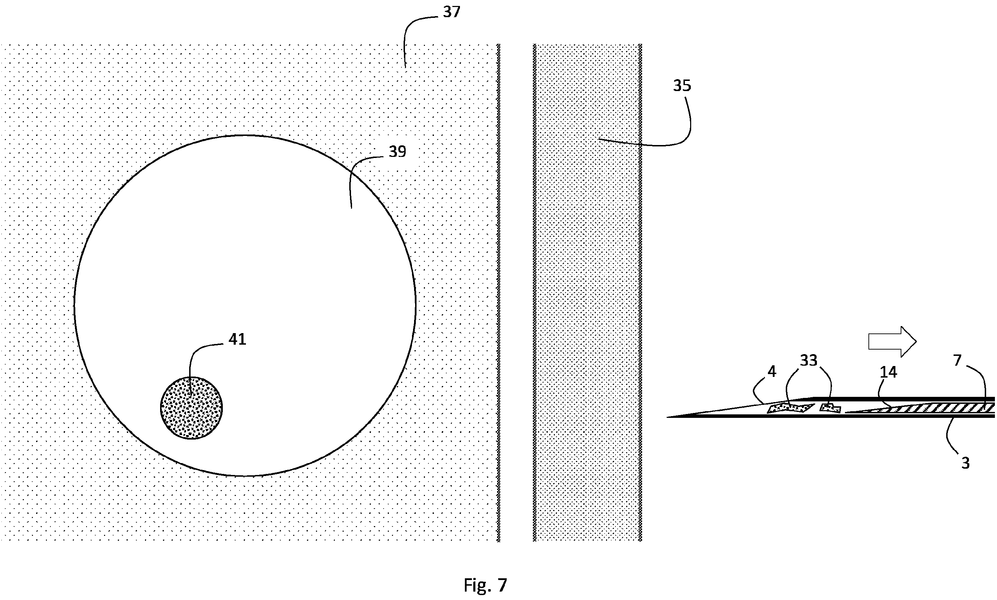

Auch beim finalen Herausziehen des Biopsieelements 3 aus dem Körper des Patienten kann, wie in Fig. 7 gezeigt, darauf geachtet werden, dass das Fluoreszenz-Signal auffällig genug bleibt. So kann beispielsweise verhindert werden, dass sich nicht vollständig abgeschilfertes Gewebe beim Herausziehen wieder aus dem Aufnahmeraum 31 unbemerkt herauszieht.

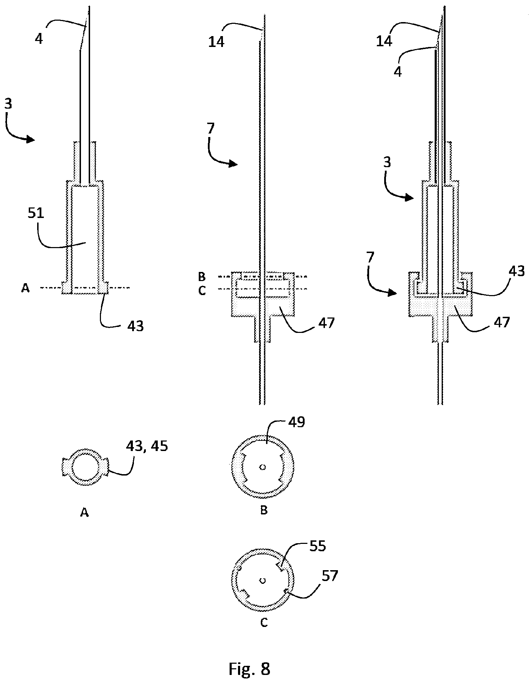

In Fig. 8 ist eine erste Ausführungsform für eine axiale Arretierung des distalen Abschnitts 7 des Fluoreszenz-Endoskopieelements 5 in der distalen Position relativ zum Biopsieelement 3 gezeigt. Dazu weist das Biopsieelement 3 proximalseitig einen Verriegelungsflansch 43 auf. In der Querschnittsansicht A wird deutlich, dass der Verriegelungsflansch 43 nicht gleichmäßig umlaufend ausgestaltet ist, sondern hier aus zwei Längsstegen 45 gebildet wird. Der distale Abschnitt 7 des Fluoreszenz-Endoskopieelements 5 weist ein zum Verriegelungsflansch 43 komplementäres weibliches Aufnahmeteil 47 auf, das in seiner axialen Position abgestimmt auf die Länge des Biopsieelements 3 ist. Das weibliche Aufnahmeteil 43 hat in den beiden Querschnittsansichten B, C eine jeweils unterschiedliche Querschnittskontur mit jeweils unterschiedlich in Umfangsrichtung positionierten Längsnuten 49, durch welche die Längsstege 45 des Biopsieelements 3 passen. Durch entsprechende Drehstellung kann der Verriegelungsflansch 43 damit in das Aufnahmeteil 47 eingeführt und mittels Verdrehung darin verriegelt werden. Vorzugsweise sind der Verriegelungsflansch 43 und das Aufnahmeteil 47 für die sichere, dichte und stabile mechanische Kopplung mit einem Luer-System kombiniert, das einen Innenkegel 51 (hier vereinfacht als Zylinder gezeigt) und einen korrespondierenden Außenkegel (einer hier nicht gezeigten anderen Komponente) aufweist. Das Luer-System ist vorzugsweise standardisiert und erlaubt die Verwendung von bereits verfügbaren Biopsieelementen. Die axiale Position des Aufnahmeteils 47 bezüglich des distalen Abschnitts 7 des Fluoreszenz-Endoskopieelements 5 ist so festgelegt, dass bei aufgenommenem und verriegeltem Verriegelungsflansch 43 das distale Ende 14 des distalen Abschnitts 7 des Fluoreszenz-Endoskopieelements 5 bündig mit der distalen Spitze 4 des Biopsieelements 3 abschließt. In der Querschnittskontur C sind Radialanschläge 55 vorgesehen, damit die Drehstellung der abgeschrägten distalen Spitze 4 des Biopsieelements 3 und des analog abgeschrägten distalen Endes 14 des distalen Abschnitts 7 des Fluoreszenz-Endoskopieelements 5 aufeinander abgestimmt sind. Eine zusätzliche Rastfunktion, z.B. durch Rastnasen 57 an einer Innenseite des Aufnahmeteils 47, kann verhindern, dass sich das Biopsieelement 3 und der distaler Abschnitt 7 des Fluoreszenz-Endoskopieelements 5 bei der Anwendung um ihre gemeinsame Längsachse gegeneinander verdrehen. Die Längsstege 45 liegen nämlich eingerastet zwischen den Rastnasen 57 und den Radialanschlägen 55. Der distale Abschnitt 7 des Fluoreszenz-Endoskopieelements 5 ist also in der distalen Position drehfest um seine Längsachse im Biopsieelement 3 in einer definierten Drehstellung gelagert.

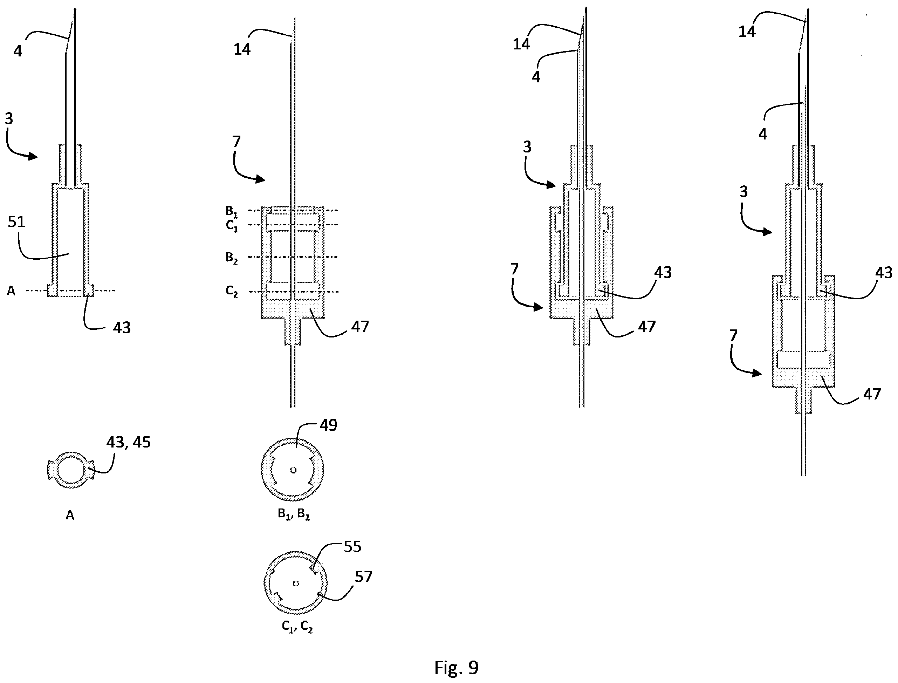

In Fig. 9 ist eine zweite Ausführungsform mit axialen Arretierungen des distalen Abschnitts 7 des Fluoreszenz-Endoskopieelements 5 sowohl in der distalen Position als auch in der proximalen Position relativ zum Biopsieelement 3 gezeigt. Im Gegensatz zur ersten Ausführungsform gemäß Fig. 8 ist die proximale Position in der zweiten Ausführungsform nicht beliebig wählbar, sondern strukturell festgelegt. Das Biopsieelement 3 unterscheidet sich dabei nicht vom Biopsieelement 3 gemäß Fig. 8, d.h. es weist ebenfalls proximalseitig den Verriegelungsflansch 43 auf. Das zum Verriegelungsflansch 43 komplementäre weibliche Aufnahmeteil 47 des Fluoreszenz-Endoskopieelements 5 weist Querschnittskonturen B₁, B₂ und C₁, C₂ an axial unterschiedlichen Positionen auf. Die Querschnittskontur C₁ definiert dabei die distale Position und die Querschnittskontur C₂ definiert die proximale Position. Die Querschnittskonturen B₁, B₂ dienen der Verriegelung des Verriegelungsflansches 43 in den jeweiligen axialen Positionen C₁ und C₂. Analog zu Fig. 8 kann der Verriegelungsflansch 43 durch entsprechende Drehstellung in das Aufnahmeteil 47 durch die Querschnittskonturen B₁, B₂ axial bewegt und mittels Verdrehung in den axialen Positionen C₁ und C₂ verriegelt werden. Die axiale Position der Querschnittskontur C₂ bezüglich des distalen Abschnitts 7 des Fluoreszenz-Endoskopieelements 5 ist so eingestellt, dass bei aufgenommenem und in der Querschnittskontur C₂ verriegeltem Verriegelungsflansch 43 das distale Ende 14 des distalen Abschnitts 7 des Fluoreszenz-Endoskopieelements 5 bündig mit der distalen Spitze 4 des Biopsieelements 3 abschließt. Der distale Abschnitt 7 des Fluoreszenz-Endoskopieelements 5 ist analog zu in Fig. 8 mittels Rastnasen 57 und Radialanschlägen 55 sowohl in der distalen Position C₂ als auch in der proximalen Position C₁ drehfest um seine Längsachse im Biopsieelement 3 in einer definierten Drehstellung gelagert.

Die nummerierten Bezeichnungen der Bauteile oder Bewegungsrichtungen als "erste", "zweite", "dritte" usw. sind hierin rein willkürlich zur Unterscheidung der Bauteile oder Bewegungsrichtungen untereinander gewählt und können beliebig anders gewählt werden. Es ist damit kein Bedeutungsrang verbunden. Eine Bezeichnung eines Bauteils oder technischen Merkmals als "erstes" soll nicht dahingehend missverstanden werden, dass es ein zweites Bauteil oder technisches Merkmal dieser Art geben muss. Außerdem können etwaige Verfahrensschritte, soweit nicht explizit anders erläutert oder zwingend erforderlich, in beliebiger Reihenfolge und/oder zeitlich teilweise oder ganz überlappend durchgeführt werden.

Äquivalente Ausführungsformen der hierin beschriebenen Parameter, Bauteile oder Funktionen, die in Anbetracht dieser Beschreibung einer fachlich versierten Person als offensichtlich erscheinen, seien hierin so erfasst als wären sie explizit beschrieben. Entsprechend soll der Schutzbereich der Erfindung solche äquivalente Ausführungsformen umfassen, sofern sie unter den Umfang der Ansprüche fallen. Als optional, vorteilhaft, bevorzugt, erwünscht oder ähnlich bezeichnete "kann"-Merkmale sind als optional zu verstehen und nicht als schutzbereichsbeschränkend.

Die beschriebenen Ausführungsformen sind als illustrative Beispiele zu verstehen und stellen keine abschließende Liste von möglichen Ausführungsformen dar. Jedes Merkmal, das im Rahmen einer Ausführungsform offenbart wurde, kann allein oder in Kombination mit einem oder mehreren anderen Merkmalen verwendet werden, unabhängig davon, in welcher Ausführungsform die Merkmale jeweils beschrieben wurden, sofern die entstehende Merkmalskombination unter den Umfang der Ansprüche fällt. Während mindestens ein Ausführungsbeispiel hierin beschrieben und gezeigt ist, seien Abwandlungen und alternative Ausführungsformen, die einer fachmännisch versierten Person in Anbetracht dieser Beschreibung als offensichtlich erscheinen, vom Schutzbereich dieser Offenbarung mit erfasst, sofern diese Abwandlungen und alternativen Ausführungsformen unter den Umfang der Ansprüche fallen. Im Übrigen soll hierin weder der Begriff "aufweisen" zusätzliche andere Merkmale oder Verfahrensschritte ausschließen noch soll "ein" oder "eine" eine Mehrzahl ausschließen.

### Bezugszeichenliste:

- 1: Biospiesystem
- 3: Biopsieelement
- 4: distale Spitze des Biopsieelements
- 5: Fluoreszenz-Endoskopieelement
- 7: Lichtleiter bzw. distaler Abschnitt des Fluoreszenz-Endoskopieelements
- 9: Handstück
- 11: Bedieneinheit
- 13: optische Komponente
- 14: distales Ende des distalen Abschnitts des Fluoreszenz-Endoskopieelements bzw. des Lichtleiters
- 15: Strahlteiler
- 17: Langpassfilter
- 19: Bildsensor, Photodiode, o.ä.
- 21: Lichtquelle
- 23: Kabelverbindung
- 25: Anzeigekomponente
- 27: Steuer- und Versorgungskomponente
- 29: Bedienungskomponente
- 31: Aufnahmeraum
- 33: Gewebeprobe
- 35: Haut des Patienten
- 37: Organ des Patienten
- 39: Verschattung
- 41: malignes Gewebe
- 43: Verriegelungsflansch
- 45: Längsstege
- 47: Aufnahmeteil
- 49: Längsnuten
- 51: Innenkegel
- 55: Radialanschläge
- 57: Rastnasen

## Patentansprüche

1. Biopsiesystem (1) mit
- einem Biopsieelement (3) zur Entnahme einer Probe (33) von zu untersuchendem Gewebe (41) aus einem organischen Körper,
- einem Fluoreszenz-Endoskopieelement (5),
wobei das Fluoreszenz-Endoskopieelement (5) einen distalen Abschnitt (7) mit einer Auskopplung von Anregungslicht sowie eine Einkopplung von Fluoreszenzlicht aufweist,
wobei der distale Abschnitt (7) des Fluoreszenz-Endoskopieelements (5) im Biopsieelement (3) in einer distalen Position und in einer proximalen Position axial positionierbar ist,
wobei das Biopsieelement (3) zur Aufnahme der Probe (33) des zu untersuchenden Gewebes (41) einen Aufnahmeraum (31) aufweist, welcher in der distalen Position des distalen Abschnitts (7) des Fluoreszenz-Endoskopieelements (5) distalseitig verschlossen ist und in der proximalen Position des distalen Abschnitts (7) des Fluoreszenz-Endoskopieelements (5) distalseitig geöffnet ist, **dadurch gekennzeichnet, dass** der distale Abschnitt (7) des Fluoreszenz-Endoskopieelements (5) radial dichtend in dem Biopsieelement (3) geführt ist, sodass im Aufnahmeraum (31) während eines proximalwärtigen Zurückziehens des distalen Abschnitts (7) des Fluoreszenz-Endoskopieelements (5) im Biopsieelement (3) ein Einsaugeffekt in den Aufnahmeraum (31) erzeugt wird.

2. Biopsiesystem (1) nach Anspruch 1, wobei der distale Abschnitt (7) des Fluoreszenz-Endoskopieelements (5) bei distaler Position den Aufnahmeraum (31) vollständig ausfüllt.

3. Biopsiesystem (1) nach Anspruch 1 oder 2, wobei das Fluoreszenz-Endoskopieelement (5) dazu eingerichtet ist, wahlweise bei der distalen Position des distalen Abschnitts (7) des Fluoreszenz-Endoskopieelements (5), der proximalen Position des distalen Abschnitts (7) des Fluoreszenz-Endoskopieelements (5) und/oder einer beliebigen dazwischenliegenden Position des distalen Abschnitts (7) des Fluoreszenz-Endoskopieelements (27) distalseitig Anregungslicht auszukoppeln und Fluoreszenzlicht einzukoppeln.

4. Biopsiesystem (1) nach einem der vorhergehenden Ansprüche, wobei ein distales Ende (14) des distalen Abschnitts (7) des Fluoreszenz-Endoskopieelements (5) in der distalen Position bündig mit einer distalen Spitze (4) des Biopsieelements (3) den Aufnahmeraum (31) abschließt oder distal aus der distalen Spitze (4) des Biopsieelements (15) vorsteht.

5. Biopsiesystem (1) nach einem der vorhergehenden Ansprüche, wobei der distale Abschnitt (7) des Fluoreszenz-Endoskopieelements (5) in der distalen Position und/oder in der proximalen Position relativ zum Biopsieelement (3) arretierbar ist.

6. Biopsiesystem (1) nach einem der vorhergehenden Ansprüche, wobei der distale Abschnitt (7) des Fluoreszenz-Endoskopieelements (5) zumindest in der distalen Position drehfest um seine Längsachse im Biopsieelement (3) in einer definierten Drehstellung gelagert ist.

7. Biopsiesystem (1) nach einem der vorhergehenden Ansprüche, wobei ein distales Ende (14) des distalen Abschnitts (7) des Fluoreszenz-Endoskopieelements (5) und eine distale Spitze (4) des Biopsieelements (3) in einem gleichen Winkel (γ) abgeschrägt sind.

8. Biopsiesystem (1) nach Anspruch 7, wobei der Winkel (γ) spitz ist und vorzugsweise 45° oder weniger beträgt.

9. Biopsiesystem (1) nach einem der vorhergehenden Ansprüche, wobei der distale Abschnitt (7) des Fluoreszenz-Endoskopieelements (5) und/oder das Biopsieelement (3) für den einmaligen Gebrauch als Einwegartikel ausgestaltet sind.

10. Biopsiesystem (1) nach einem der vorhergehenden Ansprüche, wobei das Fluoreszenz-Endoskopieelement (5) einen Lichtleiter (7) mit einer proximalseitigen Einkopplung von Anregungslicht und/oder einer proximalseitigen Auskopplung von Fluoreszenzlicht aufweist, wobei ein distales Ende (14) des Lichtleiters (7) als distalseitige Auskopplung des Anregungslichts und/oder als distalseitige Einkopplung des Fluoreszenzlichts fungiert.

11. Biopsiesystem (1) nach einem der vorhergehenden Ansprüche, wobei der distale Abschnitt (7) des Fluoreszenz-Endoskopieelements (5) eine LED zur Auskopplung von Anregungslicht und/oder einen Bildsensor oder eine Photodiode zur Einkopplung von Fluoreszenzlicht aufweist.

12. Biopsiesystem (1) nach einem der vorhergehenden Ansprüche, wobei das Fluoreszenz-Endoskopieelement (5) dazu eingerichtet ist, wahlweise ein erstes Anregungslicht und ein zweites Anregungslicht proximal einzukoppeln, wobei das erste Anregungslicht im Mittel kurzwelliger ist als das zweite Anregungslicht, und wobei das Fluoreszenz-Endoskopieelement (5) dazu eingerichtet ist, wahlweise ein erstes Bild oder ein erstes Fluoreszenzsignal von einem ersten Gewebebereich aus mit dem ersten Anregungslicht angeregtem ersten Fluoreszenzlicht zu erzeugen und ein zweites Bild oder ein zweites Fluoreszenzsignal von einem zweiten Gewebebereich aus mit dem zweiten Anregungslicht angeregtem zweiten Fluoreszenzlicht zu erzeugen.

13. Biopsiesystem (19) nach einem der vorhergehenden Ansprüche, wobei das Fluoreszenz-Endoskopieelement (5) proximalseitig eine mit dem distalen Abschnitt (7) des Fluoreszenz-Endoskopieelements (5) lösbar koppelbare oder fest gekoppelte optische Komponente (13) aufweist, wobei die optische Komponente (13) einen Strahlteiler (15) mit einer Bildpfadseite und einer Beleuchtungspfadseite aufweist, wobei zum Auskoppeln des Fluoreszenzlichts ein Bildsensor (19) oder eine Photodiode mit der Bildpfadseite lösbar koppelbar oder fest gekoppelt ist, und wobei zum Einkoppeln des Anregungslichts eine Lichtquelle (21) mit der Beleuchtungspfadseite lösbar koppelbar oder fest gekoppelt ist.

14. Biopsiesystem (1) nach Anspruch 13, wobei die optische Komponente (13) ein optisches Langpassfilter (17) aufweist, welches das Fluoreszenzlicht stärker zur Bildpfadseite transmittiert als das Anregungslicht, und/oder ein optisches Kurzpassfilter aufweist, welches das Fluoreszenzlicht stärker zur Bildpfadseite reflektiert als das Anregungslicht.

15. Biopsiesystem (1) nach einem der Ansprüche 13 oder 14, wobei das Fluoreszenz-Endoskopieelement (5) eine mit dem Bildsensor (19) oder der Photodiode und/oder der Lichtquelle (21) signalverbundene oder-verbindbare Betriebseinheit (11) aufweist, wobei die Betriebseinheit (11) eine optische oder akustische Anzeigekomponente (25), eine Steuer- und Versorgungskomponente (27) und eine Bedienungskomponente (29) aufweist.

16. Biopsiesystem nach einem der Ansprüche 13 bis 15, wobei die optische Komponente (13) in einem Handstück (9) des Fluoreszenz-Endoskopieelements (5) angeordnet ist, an welches der distale Abschnitt (7) des Fluoreszenz-Endoskopieelements (5) und das Biopsieelement (3) ankoppelbar sind, wobei das Handstück (9) vorzugsweise für den mehrmaligen Gebrauch ausgestaltet ist.

17. Biopsiesystem (1) nach einem der vorhergehenden Ansprüche, wobei das Biopsiesystem ein System zur Feinnadelbiospie oder Feinnadelaspirationsbiopsie ist.

## Claims

1. A biopsy system (1) comprising:
- a biopsy element (3) for taking a sample (33) of tissue (41) to be examined from an organic body,
- a fluorescence endoscopy element (5),
wherein the fluorescence endoscopy element (5) has a distal portion (7) with a decoupling of stimulation light and coupling of fluorescence light,
wherein the distal portion (7) of the fluorescence endoscopy element (5) in the biopsy element (3) is axially positionable in a distal position and in a proximal position,
wherein for receiving the sample (33) of the tissue (41) to be examined, the biopsy element (3) has a receiving space (31) which in the distal position of the distal portion (7) of the fluorescence endoscopy element (5) is distally closed and in the proximal position of the distal portion (7) of the fluorescence endoscopy element (5) is distally open, **characterised in that** the distal portion (7) of the fluorescence endoscopy element (5) is guided in a radially sealed manner in the biopsy element (3), so that in the receiving space (31), during the pulling back of the distal portion (7) of the fluorescence endoscopy element (5) proximally in the biopsy element (3), a sucking-in effect is produced in the receiving space (31).

2. The biopsy element (1) according to claim 1, wherein the distal portion (7) of the fluorescence endoscopy element (5) completely fills the receiving space (31) in the distal position.

3. The biopsy system (1) according to claim 1 or 2, wherein the fluorescence endoscopy element (5) is configured to optionally distally decouple stimulation light and couple in fluorescence light in the distal position of the distal portion (7) of the fluorescence endoscopy element (5), the proximal position of the distal portion (7) of the fluorescence endoscopy element (5) and/or any intermediate position of the distal portion (7) of the fluorescence endoscopy element (27).

4. The biopsy system (1) according to any one of the preceding claims, wherein a distal end (14) of the distal portion (7) of the fluorescence endoscopy element (5) in the distal position closes the receiving space (31) in a flush manner with a distal tip (4) of the biopsy element (3) or distally projects from the distal tip (4) of the biopsy element (15).

5. The biopsy system (1) according to any one of the preceding claims, wherein the distal portion (7) of the fluorescence endoscopy element (5) is lockable in the distal position and/or the proximal position relative to the biopsy element (3).

6. The biopsy system (1) according to any one of the preceding claims, wherein the distal portion (7) of the fluorescence endoscopy element (5) at least in the distal position is mounted in a rotationally fixed manner in a defined rotational position about its longitudinal axis in the biopsy element (3).

7. The biopsy system (1) according to any one of the preceding claims, wherein a distal end (14) of the distal portion (7) of the fluorescence endoscopy element (5) and a distal tip (4) of the biopsy element (3) are bevelled at a same angle (γ).

8. The biopsy system (1) according to claim 7, wherein the angle (γ) is acute and preferably is 45° or less.

9. The biopsy system (1) according to any one of the preceding claims, wherein the distal portion (7) of the fluorescence endoscopy element (5) and/or the biopsy element (3) are configured as a disposable article for single use.

10. The biopsy system (1) according to any one of the preceding claims, wherein the fluorescence endoscopy element (5) comprises a light guide (7) with proximal coupling in of stimulation light and/or proximal decoupling of fluorescence light, wherein a distal end (14) of the light guide (7) distally decouples the stimulation light and/or distally couples in the fluorescence light.

11. The biopsy system (1) according to any one of the preceding claims, wherein the distal portion (7) of the fluorescence endoscopy element (5) has an LED for decoupling stimulation light and/or an image sensor or a photodiode for coupling in fluorescence light.

12. The biopsy system (1) according to any one of the preceding claims, wherein the fluorescence endoscopy element (5) is configured to selectively proximally couple in a first stimulation light and a second stimulation light, wherein the first stimulation light is on average shorter wave than the second stimulation light, and wherein the fluorescence endoscopy element (5) is configured to optionally produce a first image or a first fluorescence signal of a first tissue region from first fluorescence light stimulated with the first stimulation light and a second image or a second fluorescence signal from a second tissue region from second fluorescence light stimulated with the second stimulation light.

13. The biopsy system (19) according to any one of the preceding claims, wherein proximally, the fluorescence endoscopy element (5) comprises an optical component (13) which is detachably coupleable or fixedly coupled to the distal portion (7) of the fluorescence endoscopy element (5), wherein the optical component (13) comprises a beam splitter (15) with an image path side and an illumination path side, wherein to decouple the fluorescence light an image sensor (19) or a photodiode is detachably coupleable or fixedly coupled to the image path side, and wherein to couple in the stimulation light, a light source (21) is detachably coupleable or fixedly coupled to the illumination path side.

14. The biopsy system (1) according to claim 13, wherein the optical component (13) has an optical longpass filter (17) which transmits the fluorescence light more strongly to the image path side than the stimulation light, and/or has an optical shortpass filter which reflects the fluorescence light more strongly to the image path side than the stimulation light.

15. The biopsy system (1) according to any one of claims 13 or 14, wherein the fluorescence endoscopy element (5) has an operating unit (11) signal-connected or connectable to the image sensor (19) or the photodiode and/or the light source (21), wherein the operating unit (11) has an optical or acoustic display component (25), a control and supply component (27) and an operating component (29).

16. The biopsy system according to any one of claims 13 to 15, wherein the optical component (13) is arranged in a handpiece (9) of the fluorescence endoscopy element (5), to which the distal portion (7) of the fluorescence endoscopy element (5) and the biopsy element (3) can be coupled, wherein the handpiece (9) is preferably designed for repeated use.

17. The biopsy system (1) according to any one of the preceding claims, wherein the biopsy system is a system for fine-needle biopsy or fine-needle aspiration biopsy.

## Revendications

1. Système de biopsie (1) comprenant :
- un élément de biopsie (3) pour prélever un échantillon (33) de tissu à analyser (41) dans un corps organique,
- un élément d'endoscopie par fluorescence (5),
dans lequel l'élément d'endoscopie par fluorescence (5) présente un tronçon distal (7) avec un découplage de lumière d'excitation ainsi qu'un couplage de lumière fluorescente,
dans lequel le tronçon distal (7) de l'élément d'endoscopie par fluorescence (5) peut être positionné axialement dans l'élément de biopsie (3) dans une position distale et dans une position proximale,
dans lequel l'élément de biopsie (3) présente pour prélever l'échantillon (33) du tissu à analyser (41), un espace de réception (31) qui est fermé côté distal dans la position distale du tronçon distal (7) de l'élément d'endoscopie par fluorescence (5) et ouvert côté distal dans la position proximale du tronçon distal (7) de l'élément d'endoscopie par fluorescence (5), **caractérisé en ce que** le tronçon distal (7) de l'élément d'endoscopie par fluorescence (5) est dirigé dans l'élément de biopsie (3) en étanchéité radiale, de sorte que dans l'espace de réception (31), pendant un retrait en direction proximale du tronçon distal (7) de l'élément d'endoscopie par fluorescence (5) dans l'élément de biopsie (3), un effet d'aspiration dans l'espace de réception (31) est produit.

2. Système de biopsie (1) selon la revendication 1, dans lequel le tronçon distal (7) de l'élément d'endoscopie par fluorescence (5) remplit complètement l'espace de réception (31) en position distale.

3. Système de biopsie (1) selon la revendication 1 ou 2, dans lequel l'élément d'endoscopie par fluorescence (5) est conçu, au choix dans la position distale du tronçon distal (7) de l'élément d'endoscopie par fluorescence (5), la position proximale du tronçon distal (7) de l'élément d'endoscopie par fluorescence (5) et/ou une position intermédiaire quelconque du tronçon distal (7) de l'élément d'endoscopie par fluorescence (27), pour découpler de la lumière d'excitation et coupler de la lumière fluorescente côté distal.

4. Système de biopsie (1) selon l'une des revendications précédentes, dans lequel une extrémité distale (14) du tronçon distal (7) de l'élément d'endoscopie par fluorescence (5) dans la position distale referme l'espace de réception (31) à l'affleur avec une pointe distale (4) de l'élément de biopsie (3) ou est en saillie distale hors de la pointe distale (4) de l'élément de biopsie (15).

5. Système de biopsie (1) selon l'une des revendications précédentes, dans lequel le tronçon distal (7) de l'élément d'endoscopie par fluorescence (5) peut être bloqué dans la position distale et/ou dans la position proximale par rapport à l'élément de biopsie (3).

6. Système de biopsie (1) selon l'une des revendications précédentes, dans lequel le tronçon distal (7) de l'élément d'endoscopie par fluorescence (5) est disposé, au moins dans la position distale, fixe en rotation sur son axe longitudinal dans l'élément de biopsie (3), dans une position de rotation définie.

7. Système de biopsie (1) selon l'une des revendications précédentes, dans lequel une extrémité distale (14) du tronçon distal (7) de l'élément d'endoscopie par fluorescence (5) et une pointe distale (4) de l'élément de biopsie (3) sont biseautées dans un angle égal (γ).

8. Système de biopsie (1) selon la revendication 7, dans lequel l'angle (γ) est aigu et fait de préférence 45° ou moins.

9. Système de biopsie (1) selon l'une des revendications précédentes, dans lequel le tronçon distal (7) de l'élément d'endoscopie par fluorescence (5) et/ou l'élément de biopsie (3) sont conçus pour l'usage unique en tant qu'article jetable.

10. Système de biopsie (1) selon l'une des revendications précédentes, dans lequel l'élément d'endoscopie par fluorescence (5) présente un conduit de lumière (7) avec un couplage de lumière d'excitation côté proximal et/ou un découplage de lumière fluorescente côté proximal, dans lequel une extrémité distale (14) du conduit de lumière (7) sert en tant que découplage côté distal de la lumière d'excitation et/ou en tant que couplage côté distal de la lumière fluorescente.

11. Système de biopsie (1) selon l'une des revendications précédentes, dans lequel le tronçon distal (7) de l'élément d'endoscopie par fluorescence (5) présente une LED pour le découplage de la lumière d'excitation et/ou un capteur d'images ou une photodiode pour le couplage de lumière fluorescente.

12. Système de biopsie (1) selon l'une des revendications précédentes, dans lequel l'élément d'endoscopie par fluorescence (5) est conçu pour coupler au choix une première lumière d'excitation et une seconde lumière d'excitation, dans lequel la première lumière d'excitation a des ondes en moyenne plus courtes que la seconde lumière d'excitation, et dans lequel l'élément d'endoscopie par fluorescence (5) est conçu pour produire au choix une première image ou un premier signal de fluorescence d'une première zone tissulaire avec la première lumière fluorescente excitée par la première lumière d'excitation et une seconde image ou un second signal de fluorescence d'une seconde zone tissulaire avec la seconde lumière fluorescente excitée par la seconde lumière d'excitation.

13. Système de biopsie (19) selon l'une des revendications précédentes, dans lequel l'élément d'endoscopie par fluorescence (5) présente côté proximal une composante optique (13) pouvant être couplée de manière séparable ou couplée fixement au tronçon distal (7) de l'élément d'endoscopie par fluorescence (5), dans lequel la composante optique (13) présente un séparateur de faisceau (15) avec un côté de chemin d'image et un côté de chemin d'éclairage, dans lequel pour découpler la lumière fluorescente, un capteur d'image (19) ou une photodiode peut être couplé(e) de manière séparable ou est couplé(e) fixement au côté de chemin d'image, et dans lequel pour coupler la lumière d'excitation, une source de lumière (21) peut être couplée de manière séparable ou est couplée fixement au côté de chemin d'éclairage.

14. Système de biopsie (1) selon la revendication 13, dans lequel la composante optique (13) est un filtre passe-haut (17) optique qui transmet la lumière fluorescente plus fortement vers le côté de chemin d'image que la lumière d'excitation, et/ou présente un filtre passe-bas optique qui réfléchit la lumière fluorescente plus fortement vers le côté de chemin d'image que la lumière d'excitation.

15. Système de biopsie (1) selon l'une des revendications 13 ou 14, dans lequel l'élément d'endoscopie par fluorescence (5) présente une unité fonctionnelle (11) reliée ou pouvant être reliée en signal avec le capteur d'image (19) ou la photodiode et/ou la source de lumière (21), dans lequel l'unité fonctionnelle (11) présente une composante d'affichage (25) optique ou acoustique, une composante de commande et d'alimentation (27) et une composante de manipulation (29).

16. Système de biopsie selon l'une des revendications 13 à 15, dans lequel la composante optique (13) est disposée dans une pièce à main (9) de l'élément d'endoscopie par fluorescence (5) à laquelle le tronçon distal (7) de l'élément d'endoscopie par fluorescence (5) ou l'élément de biopsie (3) peuvent être couplés, dans lequel la pièce à main (9) est conçue de préférence pour l'usage unique.

17. Système de biopsie (1) selon l'une des revendications précédentes, dans lequel le système de biopsie est un système pour la biopsie à l'aiguille fine ou biopsie par aspiration à l'aiguille fine.
